Europäisches Patentamt

European Patent Office

Office européen des brevets

(19)

(11) Veröffentlichungsnummer: **0 326 674**
**A2**

(12) **EUROPÄISCHE PATENTANMELDUNG**

(21) Anmeldenummer: **88120105.7**

(22) Anmeldetag: **02.12.88**

(51) Int. Cl.⁴: **C07C 31/125 , C07C 29/17 , B01J 23/86**

(30) Priorität: **05.02.88 DE 3803464**

(43) Veröffentlichungstag der Anmeldung:
**09.08.89 Patentblatt 89/32**

(84) Benannte Vertragsstaaten:
**BE DE FR IT NL SE**

(71) Anmelder: **HÜLS AKTIENGESELLSCHAFT**
**- RSP Patente / PB 15 - Postfach 13 20**
**D-4370 Marl 1(DE)**

(72) Erfinder: **Lüken, Hans-Gerd, Dr.**
**Droste-Hülshoff-Strasse 123**
**D-4370 Marl(DE)**
Erfinder: **Tanger, Uwe, Dr.**
**Trackener Strasse 2**
**D-4630 Bochum(DE)**
Erfinder: **Droste, Wilhelm, Dr.**
**Pommernstrasse 4 a**
**D-4370 Marl(DE)**
Erfinder: **Ludwig, Gerhard, Dr.**
**Holtweg 23**
**D-4358 Haltern(DE)**
Erfinder: **Gubisch, Dietmar, Dr.**
**Distelbeckhof 163**
**D-4300 Essen 12(DE)**

(54) Verfahren zur Herstellung von 2-Ethylhexanol durch Hydrierung von 2-Ethylhexenal in der Flüssigphase und Katalysator zu seiner Herstellung.

(57) Die Hydrierung von 2-Ethylhexenal zu 2-Ethylhexanol in der Flüssigphase führte bisher nur zu unreinen Produkten mit hohen Bromzahlen.

Mit dem beanspruchten Trägerkatalysator ist die Hydrierung bei vergleichsweise niedrigen Temperaturen mit hoher Katalysatorbelastung möglich. Man erhält ein sehr reines 2-Ethylhexanol mit niedrigen Bromzahlen.

Herstellung des Katalysators und von 2-Ethylhexanol

EP 0 326 674 A2

# Verfahren zur Herstellung von 2-Ethylhexanol durch Hydrierung von 2-Ethylhexenal in der Flüssigphase und Katalysator zu seiner Herstellung

2-Ethylhexanol wird als Veresterungskomponente, z. B. zur Herstellung von Dioctylphthalat als Weichmacher für PVC in großen Mengen durch Hydrierung von 2-Ethylhexenal (Oxofolgeprodukt) hergestellt. Die Hydrierung kann dabei sowohl in der Gasphase (DE-AS11 52 393) als auch in der Flüssigphase (DE-AS 19 49 296) erfolgen, wobei man in der Flüssigphase i. a. infolge verbesserter Wärmeabfuhr höhere Katalysatorbelastungen erzielen kann. Neben der Katalysatorbelastung ist die Reinheit des Produktes, ausgedrückt z. B. durch die Bromzahl, von erheblichem Interesse, da die später hergestellten Weichmacher eine gute Farbstabilität aufweisen müssen. Aus der DE-AS 19 49 296 ist bekannt, daß die Flüssigphasenhydrierung von 2-Ethylhexenal mit Kreislauffahrweise des Reaktionsproduktes bei Katalysatorbelastungen von ca. 1,8/h zu einem Produkt mit einer Bromzahl zwischen 3,9 und 0,2 führt. Die hier benutzten Katalysatoren haben den Nachteil, daß bei zwar ansprechenden Katalysatorbelastungen während der Hydrierung ein von der Bromzahl her unzureichendes Produkt entsteht, dessen Reinheitsgrad durch eine nachfolgende Destillation verbessert werden muß.

Weiterhin ist aus der DE-AS 12 69 605 ein Verfahren bekannt, in dem in flüssiger Phase bei erhöhten Drücken und erhöhten Temperaturen die Umsetzung von 2-Ethylhexenal an Nickel- und/oder Kobaltkatalysatoren mit Zusätzen an Kupfer und/oder Chrom und/oder Mangan erfolgt. Ein wesentlicher Teil der Katalysatoren ist der weitere Zusatz einer Pyro- oder Polysäure in freier Form und/oder in Form mindestens eines Salzes der oben angeführten Metalle.
Nachteilig für das Verfahren und die Katalysatoren wirkt sich die Notwendigkeit sehr hoher Drücke (vorzugsweise 200 bis 400 bar) und hoher Temperaturen aus. Des weiteren sind die Katalysatorbelastungen von ca. 0,2/h sowie die hohen Bromzahlen des Produktes von ca. 0,13 bis 0,1 ungünstig für die Hydrierung.

Hieraus ergab sich die Aufgabe, ein Verfahren zu finden, bei dem die Hydrierung bei hohen Katalysatorbelastungen zu einem 2-Ethylhexanol besonderer Reinheit mit niedrigen Bromzahlen führt.

Diese Aufgabe wird erfindungsgemäß entsprechend den Angaben der Patentansprüche gelöst.

Es wurde überraschenderweise gefunden, daß die Hydrierung von 2-Ethylhexenal bei hohen Katalysatorbelastungen sowie bei erhöhtem Druck und erhöhter Temperatur ein Produkt besonderer Reinheit mit niedrigen Bromzahlen ergibt, wenn man die Reaktion an Trägerkatalysatoren durchführt, die als Hauptmetallkomponenten Kupfer und Nickel und als Stabilisator Chrom, sowie anstelle saurer Zusätze, wie Pyro-oder Polysäuren, eine Alkalikomponente, die bevorzugt als Alkalidichromat auf den Katalysator gebracht werden, enthalten.

Ein unerwarteter, wesentlicher technischer Fortschritt der Erfindung ist, daß durch den Zusatz von Alkalidichromaten zum Katalysator keine Verlangsamung der Reaktionsgeschwindigkeit auftritt, wohl aber eine verbesserte Produktqualität, ausgedrückt durch die Bromzahl, erhalten wird und die Reaktion auch bei vergleichsweise niedrigen Drücken erfolgen kann. Die Reaktion wird bei Temperaturen von 120 bis 180°C, bevorzugt bei 140 bis 160°C, insbesondere bei 145 bis 155°C, und bei Drücken von 10 bis 100 bar, bevorzugt bei 20 bis 50 bar, insbesondere bei 30 bis 40 bar durchgeführt. Bei dem erfindungsgemäßen Verfahren erreicht man im allgemeinen Katalysatorbelastungen von 2 bis 7/h und erhält ein sehr reines 2-Ethylhexanol mit Bromzahlen von 0,1 bis 0,01.

Hauptbestandteile der aktiven Komponenten des Katalysators sind Kupfer und Nickel in Konzentrationen von 15 bis 0,3 Gew.-% bzw. 15 bis 0,3 Gew.-%, bezogen auf den Katalysator. Als Aktivator sind dem Katalysator 0,05 bis 3,5 Gew.-% Cr sowie 0,01 bis 1,6 Gew.-%, vorzugsweise 0,02 bis 1,2 Gew.-% Alkali in Form von Alkalidichromat auf den Katalysator aufgebracht, zugesetzt. Als Alkalidichromat ist vorzugsweise Na-dichromat geeignet. Als Katalysatorträger setzt man bevorzugt $SiO_2$ sowie $Al_2O_3$ ein.

Die aktiven Bestandteile des Katalysators können sowohl homogen als auch in Form einer Randzone in den Katalysatorträgern verteilt sein.

Die Herstellung des Katalysators erfolgt durch Sprühimprägnierung. Falls der Katalysator eine homogene Verteilung der aktiven Komponenten im Träger enthalten soll, wird eine die aktiven Komponenten in Form von Metallsalzen enthaltende Lösung angesetzt, die ungefähr dem 0,8-fachen Volumen des Porenvolumens der in einer Dragiertrommel vorgelegten Trägermasse entspricht. Die Metallsalzlösung, deren Metallgehalt der gewünschten Beladung des Trägers entspricht, wird dann bei Raumtemperatur auf den Träger gesprüht, wobei die Metallsalzlösung in den Träger eindringt und das Porenvolumen des Trägers entsprechend dem aufgesprühten Lösungsvolumen absättigt.

Ist ein randzonenimprägnierter Katalysator erwünscht, wird eine Metallsalzlösung auf einen vorgeheizten Träger aufgesprüht und der Träger während des Sprühvorganges weiter erwärmt. Die Metallsalze kristallisieren dann in einer z. B. von der Trägertemperatur abhängigen Schichtdicke. Geeignete Temperaturen sind beispielsweise 90 bis 100 °C. Vor dem Einsatz wird der Katalysator mit Wasserstoff reduziert.

## Beispiel 1

Zur Herstellung der Imprägnierlösung werden 37g Kupfernitrat • $3H_2O$, 857,2 g Ni$(NO_3)_2$-Lösung mit 14 % Ni, 5,7g Natriumdichromat • $2H_2O$ und 1,4 g 65%ige Salpetersäure in Wasser eingebracht und gelöst. Nach Filtration ergeben sich nach Zugabe von Wasser 4 336 g Lösung. Die wäßrige Metallsalzlösung wird bei einer Trägertemperatur von 98 °C auf 1 899 g SiO2-Perlen aufgesprüht.
Der mit Metallsalz beladene, feuchte Träger wird bei 110 °C getrocknet und bei 350 °C calciniert. Die Randzonendicke beträgt 0,24mm. Der Katalysator enthält 0,3 % Cu, 4,5 % Ni, 0,07 % Cr und 0,03 % Na. Die Reduktion von 90 g des in einem isothermen Rohrreaktor befindlichen Katalysators erfolgt bei 200 °C mit Wasserstoff. Zum Einfahren in den Gleichgewichtszustand des Katalysators werden zum Kontakt stündlich 0,5 1 2-Ethylhexenal über einen Zeitraum von 48 h bei einer Temperatur von 150 °C und einem Druck von 40 bar zudosiert. Anschließend erfolgt die Ermittlung der Katalysatoraktivität in Kreislauffahrweise. Dazu werden dem Katalysator unter den genannten Temperatur- und Druckbedingungen im Verhältnis von 3 : 1 2-Ethylhexanol und Roh-2-Ethylhexenal zugesetzt. Die Querschnittsbelastung an flüssigem Material beträgt 36 m³/m²/h. Über einen Zeitraum von 6 h wird die Bil dungsgeschwindigkeit des 2-Ethylhexanols verfolgt. Als LHSV (liquid hourly space velocity) errechnet sich für einen Umsatz von 99,9 % ein Wert von 2,1/h. Die Bromzahl des 2-Ethylhexanols beträgt 0,054.

## Beispiel 2

Zur Herstellung der Imprägnierlösung werden 137 g Nickelnitrat • $6H_2O$, 199 g Kupfernitrat • $3H_2O$, 11,2 g Natriumdichromat • $2H_2O$ und 2,8 g 65%ige Salpetersäure derart in Wasser gelöst, daß eine Metallsalzlösung eines Volumens von 928 cm³ entsteht. Die Lösung wird bei Raumtemperatur auf 1 744 g Al2O3-Granulat gesprüht.
Trocknung und Calcinierung (bei 450 °C) verlaufen gemäß Beispiel 1. Der calcinierte Katalysator enthält die aktiven Komponenten homogen im Träger verteilt. Als Metallgehalte werden 3 % Cu, 1,3 % Ni, 0,26 % Cr und 0,11 % Na gefunden. Aus den Daten des Aktivitätstests gemäß Beispiel 1 wird eine LHSV für einen Umsatz von 99,9 % von 3,3/h errechnet. Die Bromzahl des 2-Ethylhexanols beträgt 0,04.

## Beispiel 3

Analog Beispiel 2 wird ein Katalysator mit 6,5 % Cu, 3,1 % Ni, 0,6 % Cr und 0,24 % Na hergestellt und getestet. Als LHSV ergibt sich für einen Umsatz von 99,9 % 6,8/h, die Bromzahl des 2-Ethylhexanols beträgt 0,018.

## Beispiel 4

Analog Beispiel 1 wird ein Katalysator mit 5,3 % Cu, 5,4 % Ni, 2,5% Cr und 1,1 % Na hergestellt und getestet. Als LHSV ergibt sich für einen Umsatz von 99,9 % 1,2/h, die Bromzahl des 2-Ethylhexanols beträgt 0,055.

## Vergleichsbeispiel

Analog Beispiel 1 wird ein Katalysator mit 5,8 % Cu, 5,6 % Ni und 2,9% Cr hergestellt und getestet. Als LHSV ergibt sich für einen Umsatz von 99,9 % 1,1/h, die Bromzahl des Produktes beträgt 0,15.

## Ansprüche

1. Verfahren zur Herstellung von 2-Ethylhexanol durch Hydrierung von 2-Ethylhexenal in der Flüssigphase an einem Ni-Katalysator, dadurch gekennzeichnet,
daß man 2-Ethylhexenal bei Temperaturen von 120 bis 180 °C und Drücken von 10 bis 100 bar an einem Trägerkatalysator hydriert, der als aktive Komponenten Nickel und Kupfer in Konzentrationen von jeweils 15 bis 0,3 Gew.-%, bezogen auf den Katalysator, Chrom in Konzentrationen von 0,05 bis 3,5 Gew.-% und eine Alkalimetallkomponente in Konzentrationen von 0,01 bis 1,6 Gew.-%, jeweils bezogen auf den Katalysator, enthält.
2. Verfahren nach Anspruch 1, dadurch gekennzeichnet, daß man bei Temperaturen von 140 bis 160 °C, bevorzugt bei 145 bis 155 °C, hydriert.
3. Verfahren nach Anspruch 1 und 2, dadurch gekennzeichnet, daß man bei Drücken von 20 bis 50 bar, bevorzugt von 30 bis 40 bar, hydriert.
4. Verfahren nach einem der Ansprüche 1 bis 3, dadurch gekennzeichnet, daß man die Alkalikomponente über das entsprechende Dichromatsalz einbringt.

5. Verfahren nach einem der Ansprüche 1 bis 4, dadurch gekennzeichnet, daß man als Träger $SiO_2$ oder $Al_2O_3$ einsetzt.

6. Verfahren nach einem der Ansprüche 1 bis 5, dadurch gekennzeichnet, daß man die aktiven Komponenten homogen im Träger verteilt. 2

7. Verfahren nach einem der Ansprüche 1 bis 5, dadurch gekennzeichnet, daß man die aktiven Komponenten in einer Randzone des Trägers verteilt.

8. Trägerkatalysator zur Hydrierung von 2-Ethylhexenal zu 2-Ethylhexanol in der Flüssigphase, dadurch gekennzeichnet, daß der Katalysator die aktiven Komponenten Nikkel und Kupfer in Konzentrationen von jeweils 15 bis 0,3 Gew.-%, bezogen auf den Katalysator, Chrom in Konzentrationen von 0,05 bis 3,5 Gew.-% und eine Alkalimetallkomponente in Konzentrationen von 0,01 bis 1,6 Gew.-%, jeweils bezogen auf den Katalysator, enthält, wobei man die Alkalikomponente über das entsprechende Dichromatsalz einbringt.

9. Trägerkatalysator nach Anspruch 8, dadurch gekennzeichnet, daß er als Träger $SiO_2$ oder $Al_2O_3$ enthält.

10. Trägerkatalysator nach Anspruch 8 und 9, dadurch gekennzeichnet, daß die aktiven Komponenten homogen im Träger verteilt sind.

11. Trägerkatalysator nach Anspruch 8 und 9, dadurch gekennzeichnet, daß die aktiven Komponenten in einer Randzone des Trägers verteilt sind.